# EUROPEAN PATENT SPECIFICATION

(11) **EP 0 510 318 B1**
(45) Date of publication and mention of the grant of the patent: **13.12.1995**
(21) Application number: 92102479.0
(22) Date of filing: 14.02.1992
(51) Int. Cl.: A61K 7/48, A61K 35/80

(54) **Compositions for the topical treatment of burns**
Mittel zur topischen Behandlung von Verbrennungen
Compositions pour le traitement topique de brûlures

(30) Priority: 23.04.1991 IT MI911122
(43) Date of publication of application: 28.10.1992
(73) Proprietor: Nucera, Antonio Carmelo, S. Fedele Intelvi, Como (IT)
(72) Inventor: Nucera, Antonio Carmelo, S. Fedele Intelvi, Como (IT)
(74) Representative: Klausner, Erich

(56) References cited:
- FR-A- 1 271 520
- STN FILE SUPPLIER, KARLSRUHE DE, FILE CHEMICAL ABSTRACTS, vol. 106, no. 4, abstract no. 23305z, Columbus, Ohio, US; & RO-A-88 580 (CENTRALE INDUSTRIALA DE MEDICAMENTE, COSMETICE, COLORANTI SI LACURI) 28-02-1986
- WPIL / DERWENT, section Ch, week 8709, class D, AN=87-060416, Derwent Publications Ltd, London, GB; & JP-A-62014769 (Chuo Giken Kogyo K.K. et al.) 23-01-1987
- SPC SOAP, PERFUMERY & COSMETICS, vol. 64, no. 6, June 1991, pages 40-41,43, London, GB; A. DWECK: "Treasure chest of the deep"

## Description

The instant invention concerns compositions suitable for topically treating burns of any kind and of any extension, also sunburns.

The inventive compositions can moreover be used for preventing said sunburns. Many, more or less effective preparations - in the form of emulsions, ointments, gels or sprays - for the topical treatment of burns and/or for the prevention of sunburns already exist on the market. Nonetheless, the Applicant is not aware of there being presently marketed compositions for the topical treatment of burns providing all the advantages offered by the inventive compositions: especially the non-toxicity of the compositions, even were children to ingest them by mistake. Of particular therapeutical relevance is the immediate analgesic action of the inventive compositions.

RO-A-88 580 discloses a gel, useful for the treatment of burns, containing methylcellulose 3-5%, pectin 5-8%, alcoholic propolis extract 20-25%, and the balance super-vivum tectorum juice.

According to one embodiment, the compositions for the topical treatment of burns can be in the form of a gel containing from 1% to 50% propolis and from 99% to 50% Ahnfedltia concinna.

According to the instant invention it is moreover possible to enhance the cicatrizing and anaesthetic activity of the compositions for the topical treatment of burns by adding a therapeutically effective amount of aloe juice or extracts. An amount of from 1% to 30% active ingredient has generally shown to be adequate. The addition of aloe has also the advantage of enhancing the anaesthetic activity of the inventive compositions and of imparting to them an anti-pruritic activity. Aloe,moreover, imparts an antimicrobial activity against some microorganisms that colonize on the skin,further imparting long storeability to the inventive compositions.

The cicatrizing activity of the inventive compositions can be further enhanced by adding a therapeutically effective amount of placenta such as, e.g., of from 1% to 10%. The placenta can be added alone or in addition to the aloe. In this latter case, though, the aloe will preferably not be present in an amount exceeding 25%.The use of placenta has shown to be particularly advantageous for the more serious burns inasmuch as placenta stimulates the regeneration of the damaged tissue. If one desires to confer to the inventive compositions a protective activity against desquamation of the skin, the addition of a suitable amount of shea butter and/or jojoba such as,e.g.,of from 5% to 30%,is particularly useful.

When instead the compositions of the instant invention are meant to be used for the prevention of sunburns, said compositions should preferably contain from 1% to 5% propolis, from 5% to 10% aloe extracts,from 1% to 10% compounds known as UV absorbers or sunscreening agents and, finally, from 75% to 93% Ahnfeldtia concinna.

The compositions of the instant invention are water repellant and thus they prevent, or strongly reduce, the formation of phlyctenas,i.e. the blisters caused either by burns or by caustics.

Up till now no adverse reactions have been noticed as a result of the use of the compositions of the instant invention except for some isolated cases of hypersensitivity or of sensitization in potentially allergic subjects. In practice, the compositions of the instant invention have not caused any undesired effect even when repeatedly applied at short intervals.

In the following examples is illustrated the preparation of the inventive compositions in the form of a gel; nevertheless the preparation of said compositions in other forms such as pastes, ointments, aerosols, and others yet,obviously does not demand any effort on the part of the man skilled in the art of making pharmaceutical or cosmetic compositions and thus it falls within the scope of the instant invention.

Along the lines of the examples which follow hereinunder and on grounds of the indications given herein regarding the pharmacological activity of the various components, the skilled artisan will encounter no problem when modifying the dosage of the components in order to fit it to his needs: also in that case no inventive effort is demanded.

### Example 1 : Binary compositions

In a suitable vessel are thoroughly admixed Ahnfeldtia concinna gel and propolis, at a temperature of from 30° to 50°C in the following proportions:

| Ahnfeldtia concinna (%) | Propolis (%) |
|---|---|
| 99 | 1 |
| 95 | 5 |
| 90 | 10 |
| 85 | 15 |
| 80 | 20 |
| 75 | 25 |
| 70 | 30 |
| 65 | 35 |
| 60 | 40 |
| 55 | 45 |
| 50 | 50 |

At the end, the jelly mass is allowed to cool off to room temperature. The thus prepared compositions are all of such a consistency and fluidity as to be easily applied to burned surfaces.

The compositions with the lower propolis content (1% to 5%) are primarily indicated for the treatment of sunburns or slight burns.

### Example 2 : Cosmetic compositions

If in the compositions containing from 1% to 5% propolis, according to Example 1,from about 1% to 10% Ahnfeldtia concinna is replaced by the same amount of one or more substances capable of absorbing UV radiations having a wavelength of from 290 to 400 nm, a gel is obtained capable of efficaciously preventing sunburns.

Suitable sunscreens are available in commerce. Exemplarily the following sunscreens can be cited: p-aminobenzoic acid (PABA) and the derivatives thereof such as, e.g.,pentyl-p-dimethylaminobenzoate (padimate A) or ethylhexyl-p-methylbenzoate (padimate O); or associations on the basis of benzofenones and cinnamates. In lieu of chemical sunscreens, so-called physical sunscreens can also be used such as,e.g., zinc oxide, titanium dioxide, red petrolatum and others yet.

Finally, perfumes can obviously also be added.

### Example 3 : Aloe ternary compositions

Following the procedure described in Example 1, one admixes Ahnfeldtia concinna, propolis and aloe juice.

| Ahnfeldtia concinna (%) | propolis (%) | aloe (%) |
|---|---|---|
| 98 | 1 | 1 |
| 95 | 3 | 2 |
| 95 | 2 | 3 |
| 90 | 5 | 5 |
| 90 | 3-7 | 7-3 |
| 85 | 10 | 5 |
| 80 | 10 | 10 |
| 80 | 15 | 5 |
| 70 | 20 | 10 |
| 70 | 25-10 | 20-5 |
| 60 | 30 | 10 |
| 60 | 20 | 20 |
| 50 | 25 | 25 |
| 50 | 30 | 20 |
| 50 | 40 | 10 |

The addition of aloe juice or aloe extracts allows to further accelerate the rate of healing of burns and to sharply reduce the prurigo during the formation of the cicatrix.

### Example 4 : Placenta ternary compositions

The procedure described in Example 3 is followed using equal amounts of placenta in lieu of aloe.

The so prepared compositions are mainly distinguished by a more rapid regeneration of the damaged tissue.

### Example 5 : Compositions containing aloe and placenta

The procedure described in Example 1 is followed admixing the four components cited belowin the amounts listed hereinunder:

| Ahnfeldtia concinna (%) | propolis (%) | aloe (%) | placenta (%) |
|---|---|---|---|
| 90 | 5 | 2.5 | 2.5 |
| 80 | 10 | 5 | 5 |
| 80 | 10 | 3 | 7 |
| 70 | 20 | 5 | 5 |
| 70 | 10 | 5 | 15 |
| 70 | 10 | 15 | 5 |
| 60 | 20 | 10 | 10 |
| 60 | 30 | 5 | 5 |
| 50 | 30 | 10 | 10 |
| 50 | 20 | 10 | 20 |

The thus prepared compositions have a very marked healing capacity together with an excellent anaesthetic power as well as a high capacity of regenerating the tissues damaged by burns.

### Example 6 : 5 and 6 component compositions

If it is desired to impart both to the therapeutical as well as to the cosmetic compositions an enhanced protective activity with respect to skin desquamation and/or particular efficacy vis-à-vis the formation of blisters, it is very advantageous to replace a part of Ahnfeldtia concinna, usually of from 5% to 30%,with an equal amount of jojoba and/or shea butter. Jojoba, which - as is known - is a liquid wax, and shea butter impart water repellancy to the inventive compositions, property which proves very advantageous also for sunscreening compositions when used while bathing inasmuch as they prevent the latter from being washed off from the skin.

## Claims

1. A composition for the topical treatment of burns of any kind and extension characterized in that it contains a therapeutically effective amount of propolis in Ahnfeldtia concinna gel.

2. A composition according to Claim 1 characterized in that it contains also a therapeutically effective amount of aloe juice or aloe extracts.

3. A composition according to Claim 1 characterized in that it contains also a therapeutically effective amount of placenta.

4. A composition according to Claim 1 characterized in that it contains also a therapeutically effective amount of aloe juice or aloe extracts and a therapeutically effective amount of placenta.

5. A composition according to Claim 4 characterized in that an amount of from 5%to 30% Ahnfeldtia concinna is replaced by an equal amount of jojoba and/or shea butter.

6. A cosmetic composition for the prevention of sunburns characterized in that it contains from 1% to 5% propolis,from 1% to 10% screening agent(s) and from 98% to 85% Ahnfeldtia concinna.

## Patentansprüche

1. Zusammensetzung für die topische Behandlung von Verbrennungen jeder Art und Ausdehnung, dadurch gekennzeichnet, daß sie eine therapeutisch wirksame Menge an Propolis in Ahnfeldtia concinna Gel enthält.

2. Zusammensetzung nach Anspruch 1, dadurch gekennzeichnet, daß sie auch eine therapeutisch wirksame Menge an Aloe-Saft oder Aloe-Extrakten enthält.

3. Zusammensetzung nach Anspruch 1, dadurch gekennzeichnet, daß sie auch eine therapeutisch wirksame Menge an Placenta enthält.

4. Zusammensetzung nach Anspruch 1, dadurch gekennzeichnet, daß sie auch eine therapeutisch wirksame Menge Aloe-Saft oder Aloe-Extrakte und eine therapeutisch wirksame Menge an Placenta enthält.

5. Zusammensetzung nach Anspruch 4, dadurch gekennzeichnet, daß eine Menge von 5 % bis 30 % Ahnfeldtia concinna durch eine gleiche Menge an Jojoba und/oder Sheabutter ersetzt wird.

6. Kosmetische Zusammensetzung zur Verhütung von Sonnenbrand, dadurch gekennzeichnet, daß sie von 1 bis 5 % Propolis, von 1 bis 10 % Schutzstoff(e) und von 98 bis 85 % Ahnfeldtia concinna enthält.

## Revendications

1. Composition pour le traitement topique de tout type de brûlures et extensions, caractérisé en ce qu'elle contient une quantité thérapeutiquement efficace de propolis dans un gel Ahnfeldtia concinna.

2. Composition selon la revendication 1, caractérisé en ce qu'elle contient également une quantité thérapeutiquement efficace de jus d'aloès et d'extraits d'aloès.

3. Composition selon la revendication 1, caractérisé en ce qu'elle contient également une quantité thérapeutiquement efficace de placenta.

4. Composition selon la revendication 1, caractérisé en ce qu'elle contient également une quantité thérapeutiquement efficace de jus d'aloès ou d'extraits d'aloès et une quantité thérapeutiquement efficace de placenta.

5. Composition selon la revendication 4, caractérisé en qu'une quantité de 5% à 30% d'Ahnfeldtia concinna est remplacée par une quantité égale d'huile de karité et/ou de jojoba.

6. Composition cosmétique pour la prévention de brûlures solaires, caractérisée en ce q'uelle contient de 1% à 5% de propolis de 1% à 10% d'agents faisant écran et de 98% à 85% d'Ahnfeldtia concinna.
